# EUROPÄISCHE PATENTSCHRIFT

(11) **EP 0 352 674 B1**
(45) Veröffentlichungstag und Bekanntmachung des Hinweises auf die Patenterteilung: **14.04.1993**
(21) Anmeldenummer: 89113502.2
(22) Anmeldetag: 22.07.1989
(51) Int. Cl.: C07C 45/74, C07C 45/48, C07C 49/04, C07C 49/76, B01J 21/06

(54) **Verfahren zur Herstellung von Ketonen**
Process for the preparation of ketones
Procédé pour la fabrication de cétones

(30) Priorität: 29.07.1988 DE 3825873; 21.06.1989 DE 3920280
(43) Veröffentlichungstag der Anmeldung: 31.01.1990
(73) Patentinhaber: BASF Aktiengesellschaft, 67063 Ludwigshafen (DE)
(72) Erfinder: Schommer, Charles, Dr., D-6700 Ludwigshafen (DE); Ebel, Klaus, Dr., D-6704 Mutterstadt (DE); Dockner, Toni, Dr., D-6701 Meckenheim (DE); Irgang, Matthias, Dr., D-6900 Heidelberg (DE); Hoelderich, Wolfgang, Dr., D-6710 Frankenthal (DE); Rust, Harald, D-6730 Neustadt-Duttweiler (DE)

(56) Entgegenhaltungen:
- EP-A- 0 251 111
- EP-A- 0 266 510
- EP-A- 0 283 660
- DE-B- 1 201 323
- CHEMICAL ABSTRACTS, Band 103, Nr. 2, 15. Juli 1985, Seite 96, Spalte 2, Zusammenfassung Nr. 8004v, Columbus, Ohio, US; & JP-A-60 25 541
- METHODEN DER ORGANISCHEN CHEMIE, Band VII/2a, Teil 1, 1973, Seiten 627-633, Georg Thieme Verlag, Stuttgart, DE; HOUBEN WEYL

## Beschreibung

Die vorliegende Erfindung betrifft ein neues und verbessertes Verfahren zur Herstellung von Ketonen durch dehydratisierende Gasphasendecarboxylierung von Carbonsäuren oder Gemischen von Carbonsäuren und Ketonen an Titandioxid enthaltenden Katalysatoren, deren spezifische Oberfläche mindestens 10 m²/g beträgt.

Anstelle von Carbonsäuren lassen sich auch, wie aus Houben-Weyl, Methoden der organischen Chemie, Band VII/2a, S. 627-633, 1973 bekannt, deren Derivate einsetzen, z.B. Carbonsäureester, Nitrile und die Carbonsäureanhydride. Dies ist besonders dann von Interesse, wenn die Carbonsäuren schwer flüchtig sind oder bei der Verdampfung zur Decarboxylierung neigen.

Es ist allgemein bekannt (z.B. aus der DE-A-21 11 722) für diese Reaktion u.a. Zirkondioxid oder Thoriumdioxid enthaltende Kontakte, besonders solche, mit Aluminiumoxid als Trägermaterial, zu verwenden. Die mit diesen Katalysatoren erzielbaren Ausbeuten und deren Aktivitätsdauer waren nicht voll befriedigend.

Aus der DE-A-27 58 113 sind Katalysatoren bekannt, die aus Zirkondioxid oder Thoriumdioxid auf Anatas als Träger bestehen. Diese besitzen zwar eine bessere Langzeitaktivität, gegenüber den zuvor genannten, waren jedoch auch nicht voll befriedigend.

Ferner ist aus JP 58/13537, US-A-4,528,400 und US-A-4,570,021 die heterogenen katalysierte Umsetzung von Pivalinsäure entweder mit Aceton oder Essigsäure (EP-A-85 996) in der Gasphase an ZrO₂, CeO₂/Al₂O₃ und ThO₂/Al₂O₃ bekannt. Die verwendbaren Katalysatoren sind teuer bzw. radioaktiv und die Ausbeuten lassen zu wünschen übrig.

Der Erfindung lag daher die Aufgabe zugrunde, den Nachteilen der bekannten verfahren abzuhelfen.

Demgemäß wurde ein neues und verbessertes Verfahren zur Herstellung von Ketonen der allgemeinen Formel (I) 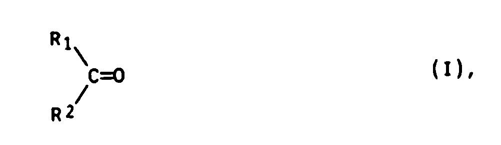
in der R¹ und R² unabhängig voneinander Alkyl, Cycloalkyl, Arylalkyl, Aryl oder Hetaryl bedeuten, wobei mindestens einer der Reste R¹ oder R² mindestens ein, d.h. ein, zwei oder drei Wasserstoffatome am α-C-Atom trägt, durch Umsetzung von zwei Carbonsäuren der allgemeinen Formeln (IIa/IIb), R¹-COOH (IIa) und R²-COOH (IIb) oder durch Umsetzung einer Carbonsäure R¹-COOH (IIa) und einem Keton 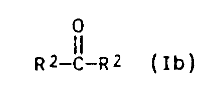
oder durch Umsetzung von Gemischen aus IIa, IIb und Ib, in der Gasphase in Gegenwart eines Katalysators gefunden, welches dadurch gekennzeichnet ist, daß man solche Katalysatoren verwendet, deren aktive Masse mindestens zu 50 Gew.-% aus Titandioxid mit einer spezifischen Oberfläche größer als 10 m²/g besteht. Der Gehalt der aktiven Masse der Katalysatoren an Titandioxid der genannten Oberfläche beträgt 50 bis 100, vorzugsweise 50 bis 99,95 Gew.-%.

Falls R¹ = R², so handelt es sich um symmetrische Ketone, in einem unsymmetrischen Keton sind R¹ und R² dagegen verschieden. Es ist jedoch auch möglich, eine Dicarbonsäure mit mindestens zwei Äquivalenten einer Monocarbonsäure zu Diketonen umzusetzen.

Die Selektivität zu den gewünschten Ketonen sind besonders hoch, wenn die spezifische Oberfläche größer als 10 m²/g, bevorzugt 20 bis 200 m²/g ist und wenn der Katalysator 0,05 bis 50 Gew.%, vorzugsweise 1 bis 10 Gew.% mindestens eines Metalloxids, ausgewählt aus der ersten oder zweiten Hauptgruppe des Periodensystems, insbesondere aus den Elementen Lithium, Natrium, Kalium oder aus der Gruppe der seltenen Erdmetalle, insbesondere aus den Elementen Lanthan oder Cer, oder Gemische dieser Oxide enthält. Das Titandioxid wird vorteilhaft in Form von Anatas verwendet.

Die erfindungsgemäßen Katalysatoren können in Form von Tränk- oder Mischkatalysatoren eingesetzt werden.

### Herstellung von Tränkkatalysatoren

Als Ausgangsmaterial wird oberflächenreiches Titandioxid, z.B. pyrogenes TiO₂ oder getrocknete Metatitansäure verwendet, das unter Zusatz von Peptisationsmitteln in einem Kneter oder Mischer in einen formbaren Zustand gebracht wird. Die Knetmasse wird extrudiert, getrocknet und calciniert. Nach Feststellung der Porosität wird eine Tränklösung eingesetzt, deren Volumen der Porenfüllung des Trägers entspricht.

Die Imprägnierung wird durch Zugabe der Tränklösung, vorteilhaft durch Aufsprühen, auf den vorgelegten Träger in einer rotierenden Trommel durchgeführt. Zur Herstellung der Tränklösungen sind alle löslichen Salze geeignet, die sich bei der Calcination ohne weitere Rückstände zu Oxiden zersetzen.

### Herstellung von Mischkatalysatoren

Die Mischkatalysatoren werden in ähnlicher Weise wie die Träger der Tränkkatalysatoren hergestellt. Zu der TiO₂-Knetmasse werden zusätzlich die entsprechenden Salzlösungen im Kneter zugegeben und es wird für gute Durchmischung gesorgt. Verformung, Trocknung und Calcinierung erfolgt wie bei der Trägerherstellung.

Die Katalysatoren lassen sich durch thermische Behandlung mit Luft oder mit Luft-Stickstoffgemischen bei 450 bis 550°C wieder regenerieren.

Der gebrauchte Katalysator kann einfach in den Schwefelsäureaufschluß zurückgeführt werden.

Nach dem erfindungsgemäßen Verfahren erhält man unter Decarboxylierung und Dehydratisierung Ketone bzw. Ketongemische

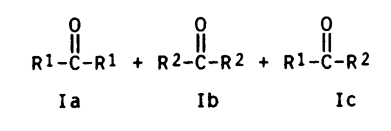

Im Falle der Herstellung von gemischten Ketonen Ic wird das statistische Mengenverhältnis von Ia : Ib : Ic = 0,25 : 0,25 : 0,50 normalerweise nicht erreicht. Wegen der unterschiedlichen Aktivitäten der Ausgangssäuren setzt sich die aktivere Säure bevorzugt mit sich selber um, so daß diese nur noch im verminderten Maß für die Bildung des gemischten Ketons zur Verfügung steht. Bei der Verwendung von Essigsäure und Isobuttersäure erhielt man nach den bekannten Verfahren beispielsweise nur 45 % des gewünschten Methylisopropylketons. Es ist ein wesentlicher Vorteil der erfindungsgemäßen Katalysatoren, daß auch die reaktionsträgere Säure stärker als im Falle herkömmlicher Katalysatoren aktiviert wird, so daß die Aktivierungsunterschiede sich verringern. Demgemäß beträgt die Ausbeute an Methylisopropylketon nach dem Verfahren der Erfindung 71 %, bezogen auf die umgesetzte Isobuttersäure bei quantitativem Umsatz.

Setzt man im Gemisch eine Carbonsäure ohne α-Wasserstoffe R¹-COOH ein, d.h. Carbonsäuren mit tert. α-C-Atom wie z.B. Pivalinsäure oder aromatische Carbonsäuren (substiuierte Benzoesäuren), so werden nur zwei Ketone Ib und Ic erhalten da R¹-COOH nicht mit sich selbst kondensiert. In diesem Falle werden mit den erfindungsgemäßen Katalysatoren Ausbeuten bis zu 99 % des gewünschten unsymmetrischen Ketons (z.B. Propiophenon) erhalten.

Die Ausbeute an gemischtem Keton läßt sich auch dadurch erhöhen, daß man die aktivere Säure in einem bis zu 10 molaren Überschuß gegenüber der weniger aktiven verwendet, allerdings erhält man in diesem Fall entsprechend große Mengen an dem symmetrischen Keton der überschüssigen Säure.

Bei der Herstellung von unsymmetrischen Ketonen Ic kann anstelle der Carbonsäure R2-COOH (IIb) ebenfalls das daraus bei der Reaktion entstehende symmetrische Keton R²-CO-R² (Ib) als Edukt eingesetzt werden, das dann nach folgender Gleichung ebenfalls zum gewünschten Keton Ic reagiert:

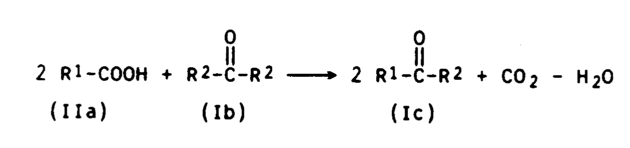

Außerdem lassen sich Gemische von Carbonsäure IIb und Keton Ib mit der Carbonsäure IIa zu I umsetzen, wobei dann nach Destillation überschussiges Ib in die Reaktion zurückgeführt werden kann. Besonders vorteilhaft läßt sich diese Variante bei der Herstellung von Methyl-ketonen wie z.B. Pinakolon, Methylisopropyl-keton und Acetophenonen anwenden, wobei dann als "Methyl-komponente" Essigsäure, Aceton oder Gemische von beiden z.B. mit Pivalinsäure, bzw. Iso-buttersäure, bzw. aromatischen Carbonsäuren umgesetzt werden.

Die Substituenten R¹ und R² bedeuten vorzugsweise eine Alkylgruppe mit 1 bis 17 C-Atomen oder eine Cycloalkylgruppe mit 3 bis 8 Ringliedern oder eine Arylalkylgruppe mit 7 bis 12 C-Atomen, eine Arylgruppe oder eine Hetarylgruppe, wobei mindestens einer der Reste R¹ oder R² mindestens ein Wasserstoffatom am α-C-Atom trägt.

Beispiele für wichtige Ketone, die als Lösungsmittel oder Zwischenprodukte für die Herstellung von Farbstoffen, Pflanzenschutzmitteln, Arzneimitteln und Vitaminen Verwendung finden, und die auch nach dem erfindungsgemäßen Verfahren aus den entsprechenden Säuren erhältlich sind, sind Diethylketon, Di-n-propylketon, Diisopropylketon, Methylpropylketon, Methylisopropylketon, Ethylisopropylketon, Nonan-5-on, Octan-2,7-dion, Cyclopentanon, Cycloheptanon, Acetophenon, Propiophenon, Butyrophenon, Isobutyrophenon, Valerophenon, Phenylaceton, 1,2-Diphenylaceton, Cyclohexylmethylketon, Cyclohexylphenylketon, Cyclopropylmethylketon, Pinacolon und sogar heterocyclische Ketone, wie 3-Acetylpyridin, 4-Acetylpyrazol, 4-Acetylimidazol.

Man kann die Carbonsäuren verwenden, die bis zu 50 Gew.% Wasser enthalten, da das Wasser sich häufig günstig auf die Aktivitätsdauer der Katalysatoren auswirkt (es scheidet sich weniger Kohlenstoff auf den Katalysator ab) ist es oft sogar zweckmäßig, die Carbonsäuren mit 1 bis 50 Gew.% Wasser zu versetzen.

Man nimmt die dehydratisierende Decarboxylierungsreaktion vorzugsweise bei Normaldruck und bei Temperaturen von 300 bis 600, vor allem 350 bis 450°C vor, indem man die auf diese Temperatur vorerhitzten Säuredämpfe durch einen Festbettofen leitet, der mit Katalysatorsträngen, -granalien, -tabletten, -splitt oder -ringen gefüllt ist oder indem man die Umsetzung in einem Wirbelofen durchführt. Im Falle schwer flüchtiger Säuren kann sich auch das Arbeiten unter vermindertem Druck empfehlen. Pro Liter Katalysator lassen sich im allgemeinen 200 bis 500 g/h der Ketone herstellen.

Nach Passieren der Katalysatorzone werden die Dämpfe gekühlt und wie üblich aufgearbeitet. Man erzielt im allgemeinen Umsätze von 97 bis 100 % und hierauf bezogene Ketonausbeuten von 55 bis 85 % bei unsymmetrischen Ketonen und 90 bis 99 % bei symmetrischen Ketonen.

### Beispiel 1

### Herstellung von Katalysatoren

Für die Herstellung der Katalysatoren wird Titandioxid mit einer Oberfläche von ca. 120 m²/g verwendet, das unter Zusatz von Wasser und 3 % Salpetersäure (bezogen auf Titandioxid) verknetet und anschließend extrudiert, getrocknet und calciniert wird. Die so erhaltenen Extrudate besitzen Porositäten von ca. 0,5 ml/g und BET-Oberflächen von ca. 100 m²/g. Die Porosität der Träger wird genau ermittelt. Die Extrudate werden bis zur Porenfüllung mit Tränklösungen in einer rotierenden Trommel imprägniert.
1.1 Auf 987 g TiO₂-Träger wird eine Lösung, die 13 g Na₂O (als wäßrige NaOH) enthält, aufgesprüht.
1.2 Auf 975 g TiO₂-Träger wird eine Lösung, die 25 g K₂O (als wäßrige KOH) enthält, aufgesprüht.

Die imprägnierten Katalysatoren werden bei 120°C 16 Stunden getrocknet und 1 Stunde bei 500°C calciniert.

### Beispiel 2

### Herstellung von Diethylketon

Über einen Verdampfer wurde stündlich ein Gemisch aus 44 g Propionsäure und 6 g Wasser verdampft und zusammen mit 3 l Stickstoff bei 360°C über 100 ml eines Katalysators geleitet, der zu 2 Gew.-% aus Kaliumoxid und dem Rest aus Anatas bestand. Anschließend wurden die Reaktionsgase abgekühlt und in einer Vorlage gesammelt. Vom zweiphasigen Austrag wurde die organische Phase mittels GC analysiert. Der Umsatz an Propionsäure betrug 100 %. Die Selektivität zu Diethylketon betrug 99 %.

### Beispiel 3

### Herstellung von Methylisopropylketon

Über einen Verdampfer wurde stündlich ein Gemisch aus 32 g Isobuttersäure, 32 g Essigsäure und 16 g Wasser verdampft und zusammen mit 3 l Stickstoff bei 420°C über 170 ml eines Katalysators geleitet, der zu 1,3 Gew.% aus Natriumoxid und dem Rest aus Anatas bestand. Anschließend wurden die Reaktionsgase abgekühlt und in einer Vorlage gesammelt. Vom zweiphasigen Austrag wurde die organische Phase mittels GC analysiert. Der Umsatz an Isobuttersäure betrug 100,0%. Die Selektivität zu Methylisopropylketon betrug 72,0 %.

### Beispiel 4

### Herstellung von Pinacolon

Über einen Verdampfer wurde stündlich ein Gemisch aus 15 g Pivalinsäure, 52 g Essigsäure und 17 g Wasser verdampft und zusammen mit 3 l Stickstoff bei 450°C über 100 ml eines Katalysators geleitet, der zu 1,3 Gew.% aus Natriumoxid und dem Rest aus Anatas bestand. Anschließend wurden die Reaktionsgase abgekühlt und in einer Vorlage gesammelt. Vom zweiphasigen Austrag wurde die organische Phase mittels GC analysiert. Der Umsatz an Pivalinsäure betrug 99 %. Die Selektivität zu Pinacolon betrug 81 %.

### Beispiel 5

### Herstellung von Propiophenon

Über einen Verdampfer wurde stündlich ein Gemisch aus 16 g Benzoesäure, 57 g Propionsäure und 14,5g Wasser verdampft und zusammen mit 10 l Stickstoff bei 400°C über 100 ml eines Katalysators geleitet, der zu 2,5 Gew.% aus Natriumoxid und dem Rest aus Anatas bestand. Anschließend wurden die Reaktionsgase abgekühlt und in einer Vorlage gesammelt. Vom zweiphasigen Austrag wurde die organische Phase mittels GC analysiert. Der Umsatz an beiden Carbonsäuren betrug jeweils 100 %. Die Selektivität zu Propiophenon betrug 99 %.

### Beispiel 6

### Herstellung von Cyclohexylphenylketon

Über einen Verdampfer wurde stündlich ein Gemisch aus 11,5 g Benzoesäure, 36 g Cyclohexancarbonsäure und 13 g Wasser verdampft und zusammen mit 10 l Stickstoff bei 450°C über 100 ml eines Katalysators geleitet, der zu 2 Gew.% aus Kaliumoxid und dem Rest aus Anatas bestand. Anschließend wurden die Reaktionsgase abgekühlt und in einer Vorlage gesammelt. Vom zweiphasigen Austrag wurde die organische Phase mittels GC analysiert. Der Umsatz an Benzoesäure betrug 98 %. Die Selektivität zu Cyclohexylphenylketon betrug 60 %.

### Beispiel 7

### Herstellung von 3-Acetylpyridin

Über einen Verdampfer wurde stündlich ein Gemisch aus 29 g Nicotinsäuremethylester, 76 g Essigsäure und 26 g Wasser verdampft und zusammen mit 10 l Stickstoff bei 420°C über 150 ml eines Katalysators geleitet, der zu 2 Gew.% aus Natriumoxid und dem Rest aus Anatas bestand. Anschließend wurden die Reaktionsgase abgekühlt und in einer Vorlage gesammelt. Vom zweiphasigen Austrag wurde die organische Phase mittels GC analysiert. Der Umsatz an Nicotinsäureester und an Essigsäure betrug jeweils 100 %. Die Selektivität zu 3-Acetylpyridin betrug 54 %. Daneben entstand Pyridin mit einer Selektivität von 41 %.

### Beispiel 8

### Herstellung weiterer Katalysatoren

### Katalysatorträger I

Es wird Titandioxid mit einer Oberfläche von ca. 120 m²/g verwendet, das unter Zusatz von Wasser und 3 % Salpetersäure (bezogen auf Titandioxid) verknetet und anschließend extrudiert, getrocknet und calciniert wird.

### Katalysatorträger II

Es wird Titandioxid mit einer Oberfläche von ca. 40 m²/g verwendet, das unter Zusatz von Wasser, 4 % Kartoffelstärke (bezogen auf Titandioxid) und 5 % Ammoniakwasser (25 % NH₃) verknetet und anschließend extrudiert, getrocknet und calciniert wird.

Die so erhaltenen Extrudate werden bis zur Porenfüllung mit Tränklösungen in einer rotierenden Trommel imprägniert:

| Katalysator | Träger | [g] | Zusatz | [g] | wäßr. Lösung von | Beispiele |
|---|---|---|---|---|---|---|
| A | II | 980 | K₂O | 20 | KOH | 2 |
| B | I | 987 | Na₂O | 13 | NaOH | 3,4 |
| C | I | 975 | Na₂O | 25 | NaOH | 5 |
| D | I | 980 | K₂O | 20 | KOH | 6 |
| E | I | 980 | Na₂O | 20 | NaOH | 7,13 |
| F | II | 980 | Li₂O | 20 | LiOH | 9 |
| G | II | 980 | Na₂O | 20 | NaOH | 10 |
| H | II | 980 | Rb₂O | 20 | Rb₂CO₃ | 11 |
| J | II | 980 | Cs₂O | 20 | Cs₂CO₃ | 12 |
| K | II | 995 | Na₂O | 5 | NaOH | 14 |
| L | I | 997 | Na₂O | 3 | NaOH | 15 |
| M | II | 999 | K₂O | 1 | KOH | 16 |
| N | II | 997 | Na₂O | 3 | NaOH | 17 |

### Beispiel 9

### Herstellung von Diethylketon

Über einen Verdampfer wurde stündlich ein Gemisch aus 43 g Propionsäure und 5 g Wasser verdampft und zusammen mit 3 l Stickstoff bei 360°C über 100 ml des Katalysators F geleitet, der zu 2 Gew.% aus Lithiumoxid und dem Rest aus Anatas bestand. Der Katalysator wurde analog Beispiel 8 durch Tränken von 980 g TiO₂-Träger II mit einer Lösung, die 20 g Li₂O (als wäßrige LiOH) enthielt, hergestellt. Anschließend wurden die Reaktionsgase abgekühlt und in einer Vorlage gesammelt. Vom zweiphasigen Austrag wurde die organsiche Phase mittels GC analysiert. Der Umsatz an Propionsäure betrug 100 %. Die Selektivität zu Diethylketon betrug 95 %.

### Beispiel 10

### Herstellung von Diethylketon

Über einen Verdampfer wurde stündlich ein Gemisch aus 53 g Propionsäure und 7 g Wasser verdampft und zusammen mit 3 l Stickstoff bei 360°C über 100 ml des Katalysators G geleitet, der zu 2 Gew.% aus Natriumoxid und dem Rest aus Anatas bestand. Der Katalysator wurde analog Beispiel 8 durch Tränken von 980 g TiO₂-Träger II mit einer Lösung, die 20 g Na₂O (als wäßrige NaOH) enthielt, hergestellt. Anschließend wurden die Reaktionsgase abgekühlt und in einer Vorlage gesammelt. Vom zweiphasigen Austrag wurde die organische Phase mittels GC analysisert. Der Umsatz an Propionsäure betrug 99 %. Die Selektivität zu Diethylketon betrug 99 %.

### Beispiel 11

### Herstellung von Diethylketon

Über einen Verdampfer wurde stündlich ein Gemisch aus 53 g Propionsäure und 7 g Wasser verdampft und zusammen mit 3 l Stickstoff bei 360°C über 100 ml des Katalysators H geleitet, der zu 2 Gew.% aus Rubidiumoxid und dem Rest aus Anatas bestand. Der Katalysator wurde analog Beispiel 8 durch Tränken von 980 g TiO₂-Träger II mit einer Lösung, die 20 g Rb₂O (als wäßrige Rb₂CO₃) enthielt, hergestellt. Anschließend wurden die Reaktionsgase abgekühlt und in einer Vorlage gesammelt. Vom zweiphasigen Austrag wurde die organische Phase mittels GC analysiert. Der Umsatz an Propionsäure betrug 100 %. Die Selektivität zu Diethylketon betrug 98 %.

### Beispiel 12

### Herstellung von Diethylketon

Über einen Verdampfer wurde stündlich ein Gemisch aus 43 g Propionsäure und 5 g Wasser verdampft und zusammen mit 3 l Stickstoff bei 360°C über 100 ml des Katalysators J geleitet, der zu 2 Gew.% aus Caesiuimoxid und dem Rest aus Anatas bestand. Der Katalysator wurde analog Beispiel 8 durch Tränken von 980 g TiO₂-Träger II mit einer Lösung, die 20 g Cs₂O (als wäßrige Cs₂CO₃) enthielt, hergestellt. Anschließend wurden die Reaktionsgase abgekühlt und in einer Vorlage gesammelt. Vom zweiphasigen Austrag wurde die organische Phase mittels GC analysiert. Der Umsatz an Propionsäure betrug 100 %. Die Selektivität zu Diethylketon betrug 97 %.

### Beispiel 13

### Herstellung von 3-Acetylpyridin

Über einen Verdampfer wurde stündlich ein Gemisch aus 22 g Nicotinsäuremethylester, 57 g Essigäsure und 20 g Wasser verdampft und zusammen mit 10 l Stickstoff bei 400°C über 100 ml des Katalysators E geleitet, der zu 2 Gew.% aus Natriumoxid und dem Rest aus Anatas bestand. Der Katalysator wurde analog Beispiel 8 durch Tränken von 980 g TiO₂-Träger I mit einer Lösung, die 20 g Na₂O (als wäßrige NaOH) enthielt, hergestellt. Anschließend wurden die Reaktionsgase abgekühlt und in einer Vorlage gesammelt. Vom zweiphasigen Austrag wurde die organische Phase mittels GC analysiert. Der Umsatz an Nicotinsäureester und an Essigsäure betrug jeweils 100 %. Die Selektivität zu 3-Acetylpyridin betrug 60 %. Daneben entstand Pyridin mit einer Selektivität von 29 %.

### Beispiel 14

### Herstellung von Pinacolon

Über einen Verdampfer wurde stündlich ein Gemisch aus 15 g Pivalinsäure, 52 g Essigsäure und 13 g Wasser verdampft und zusammen mit 3 l Stickstoff bei 415°C über 100 ml des Katalysators K geleitet, der zu 0,5 Gew.% aus Natriumoxid und dem Rest aus Anatas bestand. Der Katalysator wurde analog Beispiel 8 durch Tränken von 995 g TiO₂-Träger II mit einer Lösung, die 5g Na₂O (als wäßrige NaOH) enthielt, hergestellt. Anschließend wurden die Reaktionsgase abgekühlt und in einer Vorlage gesammelt. Vom zweiphasigen Austrag wurden die organische und die wäßrige Phase mittels GC analysiert. Der Umsatz an Pivalinsäure betrug 99 %. Die Selektivität zu Pinacolon betrug 93 %.

### Beispiel 15

### Herstellung von Pinacolon

Über einen Verdampfer wurde stündlich ein Gemisch aus 15 g Pivalinsäure, 52 g Essigsäure und 13 g Wasser verdampft und zusammen mit 3 l Stickstoff bei 400°C über 100 ml des Katalysators L geleitet, der zu 0,3 Gew.-% aus Natriumoxid und dem Rest aus Anatas bestand. Der Katalysator wurde analog Beispiel 1 durch Tränken von 997 g TiO₂-Träger I mit einer Lösung, die 3 g Na₂O (als wäßrige NaOH) enthielt, hergestellt. Anschließend wurden die Reaktionsgase abgekühlt und in einer Vorlage gesammelt. Vom zweiphasigen Austrag wurden die organische und die wäßrige Phase mittels GC analysiert. Der Umsatz an Pivalinsäure betrug 97 %. Die Selektivität zu Pinacolon betrug 95 %.

### Beispiel 16

### Herstellung von Pinacolon

Über einen Verdampfer wurde stündlich ein Gemisch aus 9 g Pivalinsäure, 32 g Aceton und 10 g Wasser verdampft und zusammen mit 3 l Stickstoff bei 410°C über 100 ml des Katalysators M geleitet, der zu 0,1 Gew.-% aus Kaliumoxid und dem Rest aus Anatas bestand. Der Katalysator wurde analog Beispiel 1 durch Tränken von 999 g TiO₂-Träger II mit einer Lösung, die 1 g K₂O (als wäßrige KOH) enthielt, hergestellt. Anschließend wurden die Reaktionsgase abgekühlt und in einer Vorlage gesammelt. Vom zweiphasigen Austrag wurden die organische und die wäßrige Phase mittels GC analysiert. Der Umsatz an Pivalinsäure betrug 98 %. Die Selektivität zu Pinacolon betrug 90 %.

### Beispiel 17

### Herstellung von Pinacolon

Über einen Verdampfer wurde stündlich ein Gemisch aus 15 g Pivalinsäure, 17 g Essigssäure, 17 g Aceton und 12 g Wasser verdampft und zusammen mit 3 l Stickstoff bei 430°C über 100 ml des Katalysators N geleitet, der zu 0,3 Gew.-% aus Natriumoxid und dem Rest aus Anatas bestand. Der Katalysator wurde analog Beispiel 1 durch Tränken von 997 g TiO₂-Träger II mit einer Lösung, die 3 g Na₂O (als wäßrige NaOH) enthielt, hergestellt. Anschließend wurden die Reaktionsgase abgekühlt und in einer Vorlage gesammelt. Vom zweiphasigen Austrag wurden die organische und die wäßrige Phase mittels GC analysiert. Der Umsatz an Pivalinsäure betrug 94 %. Die Selektivität zu Pinacolon betrug 96 %.

## Patentansprüche

1. Verfahren zur Herstellung von Ketonen der allgemeinen Formel I 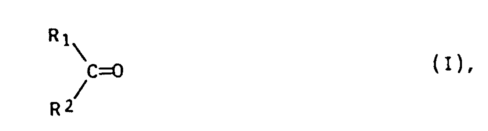 in der R¹ und R² unabhängig voneinander Alkyl, Cycloalkyl, Arylalkyl, Aryl oder Hetaryl bedeuten, wobei mindestens einer der Reste R¹ oder R² mindestens ein Wasserstoffatom am α-C-Atom trägt, durch Umsetzung von zwei Carbonsäuren der allgemeinen Formeln (IIa/IIb), R¹-COOH (IIa) und R²-COOH (IIb) oder durch Umsetzung einer Carbonsäure R¹-COOH (IIa) und einem Keton 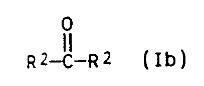 oder durch Umsetzung von Gemischen aus IIa, IIb und Ib, in der Gasphase in Gegenwart eines Katalysators, dadurch gekennzeichnet, daß man solche Katalysatoren verwendet, deren aktive Masse mindestens zu 50 Gew.-% aus Titandioxid und zu 0,05 bis 50 Gew.-% aus einem oder mehreren Metalloxiden der ersten oder zweiten Hauptgruppe des Periodensystems mit einer spezifischen Oberfläche größer als 10 m²/g besteht.

2. Verfahren nach Anspruch 1, dadurch gekennzeichnet, daß Titandioxid in Form von Anatas verwendet wird.

3. Verfahren nach Anspruch 1, dadurch gekennzeichnet, daß der Katalysator 0,05 bis 50 Gew.% Lithium-, Natrium- oder Kaliumoxid enthält.

4. Verfahren nach Anspruch 1, dadurch gekennzeichnet, daß der Katalysator ein oder mehrere Oxide seltener Erdmetalle enthält.

5. Verfahren nach Anspruch 1, dadurch gekennzeichnet, daß der Katalysator Ceroxid enthält.

6. Verfahren nach Anspruch 1, dadurch gekennzeichnet, daß der Katalysator eine spezifische Oberfläche von 20 bis 200 m²/g besitzt.

7. Verfahren nach Anspruch 1, dadurch gekennzeichnet, daß der Katalysator eine spezifische Oberfläche von 30 bis 160 m²/g besitzt.

8. Verfahren nach Anspruch 1, dadurch gekennzeichnet, daß der Katalysator 0,1 bis 10 Gew.% des Oxids enthält.

9. Verfahren nach Anspruch 1, dadurch gekennzeichnet, daß R² Methyl und R¹ tert.-Butyl bedeutet.

## Claims

1. A process for the preparation of a ketone of the formula I 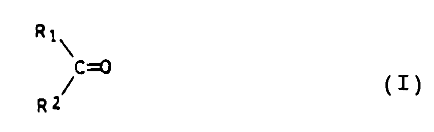 where R¹ and R² independently of one another are each alkyl, cycloalkyl, arylalkyl, aryl or hetaryl, and one or more of the radicals R¹ and R² carry one or more hydrogen atoms on the α-carbon atom, by reacting two carboxylic acids of the formulae (IIa/IIb), R¹-COOH (IIa) and R²-COOH (IIb) or by reacting a carboxylic acid R¹-COOH (IIa) and a ketone 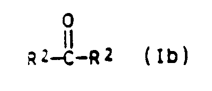 or by reacting a mixture of IIa, IIb and Ib, in the gas phase in the presence of a catalyst, wherein the catalyst used is one whose active material contains not less than 50% by weight of titanium dioxide and from 0.05 to 50% by weight of one or more metal oxides from the first or second main group of the Periodic Table having a specific surface area greater than 10 m²/g.

2. A process as claimed in claim 1, wherein titanium dioxide in the form of anatase is used.

3. A process as claimed in claim 1, wherein the catalyst contains from 0.05 to 50% by weight of lithium oxide, sodium oxide or potassium oxide.

4. A process as claimed in claim 1, wherein the catalyst contains one or more oxides of rare earth metals.

5. A process as claimed in claim 1, wherein the catalyst contains cerium oxide.

6. A process as claimed in claim 1, wherein the catalyst has a specific surface area of from 20 to 200 m²/g.

7. A process as claimed in claim 1, wherein the catalyst has a specific surface area of from 30 to 160 m²/g.

8. A process as claimed in claim 1, wherein the catalyst contains from 0.1 to 10% by weight of the oxide.

9. A process as claimed in claim 1, wherein R² is methyl and R¹ is tert-butyl.

## Revendications

1. Procédé de préparation de cétones de formule générale I 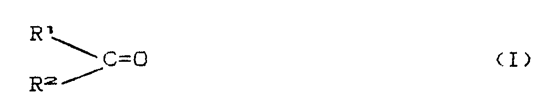 dans laquelle R¹ et R² représentent chacun, indépendamment l'un de l'autre, un reste alkyle, cycloalkyle, arylalkyle, aryle ou hétéro-aryle, l'un au moins des restes R¹ et R² portant au moins un atome d'hydrogène sur l'atome de carbone en position α, par réaction de deux acides carboxyliques de formules générales (IIa/IIb), R¹-COOH (IIa) et R²-COOH (IIb), ou par réaction d'un acide carboxylique R¹-COOH (IIa) et d'une cétone 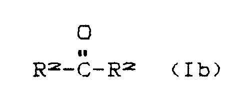 ou encore par réaction de mélanges de IIa, IIb et Ib, en phase gazeuse et en présence d'un catalyseur, caractérisé en ce qu'on utilise des catalyseurs dont la masse active se compose, au moins pour 50% en poids, de dioxyde de titane et, pour 0,05 a 50% en poids, d'un ou de plusieurs oxydes de métaux du groupe IA ou IIA du système périodique, avec une surface spécifique supérieure à 10 m²/g.

2. Procédé selon la revendication 1, caractérisé en ce qu'on utilise du dioxyde de titane sous forme d'anatase.

3. Procédé selon la revendication 1, caractérisé en ce que le catalyseur contient de 0,05 à 50% en poids d'oxyde de lithium, de sodium ou de potassium.

4. Procédé selon la revendication 1, caractérisé en ce que le catalyseur contient un ou plusieurs oxydes de métaux de terres rares.

5. Procédé selon la revendication 1, caractérisé en ce que le catalyseur contient de l'oxyde de cérium.

6. Procédé selon la revendication 1, caractérisé en ce que le catalyseur a une surface spécifique de 20 à 200 m²/g.

7. Procédé selon la revendication 1, caractérisé en ce que le catalyseur a une surface spécifique de 30 à 160 m²/g.

8. Procédé selon la revendication 1, caractérisé en ce que le catalyseur contient de 0,1 à 10% en poids de l'oxyde.

9. Procédé selon la revendication 1, caractérisé en ce que R² représente un reste méthyle et R¹ un reste tert.-butyle.
